# EUROPÄISCHE PATENTSCHRIFT

(11) **EP 1 858 566 B1**
(45) Veröffentlichungstag und Bekanntmachung des Hinweises auf die Patenterteilung: **13.04.2016**
(21) Anmeldenummer: 06706560.7
(22) Anmeldetag: 01.02.2006
(51) Int. Cl.: A61M 1/34, A61M 1/30

(54) **VORRICHTUNG ZUR ELIMINIERUNG VON SUBSTANZEN AUS FLÜSSIGKEITEN, INSBESONDERE BLUT**
DEVICE FOR THE REMOVAL OF SUBSTANCES FORM LIQUIDS IN PARTICULAR BLOOD
DISPOSITIF POUR ELIMINER DES SUBSTANCES HORS DE LIQUIDES, NOTAMMENT DU SANG

(30) Priorität: 17.02.2005 DE 102005007372
(43) Veröffentlichungstag der Anmeldung: 28.11.2007
(73) Patentinhaber: Fresenius Medical Care Deutschland GmbH, 61352 Bad Homburg v.d.H. (DE)
(72) Erfinder: CHRISTMANN, Horst, 61250 Usingen (DE)
(74) Vertreter: Laufhütte, Dieter
(86) Internationale Anmeldenummer: PCT/EP2006/000885
(87) Internationale Veröffentlichungsnummer: WO 2006/087103

(56) Entgegenhaltungen:
- EP-A- 0 472 480
- WO-A-95/18671
- DE-C1- 4 338 858
- US-A1- 2004 186 407

## Beschreibung

Die Erfindung betrifft eine Vorrichtung zur Eliminierung von Substanzen aus Flüssigkeiten, insbesondere Blut.

Es sind bereits Blutbehandlungsgeräte bekannt, bei denen Blut zunächst in einem primären extrakorporalen Kreislauf durch einen Hämodialysator oder -filter geleitet wird. Die zweite Kammer des Filters ist Teil eines Sekundärkreislaufs, in dem nur bestimmte Bestandteile des Blutes zirkuliert werden. In diesem Sekundärkreislauf sind die eigentlichen Blutbehandlungselemente, wie beispielsweise Adsorberelemente, vorgesehen. Die Aufteilung in zwei Kreisläufe kann dabei notwendig sein, wenn die Adsorberelemente nicht mit dem Vollblut, sondern mit dem Blutplasma in Verbindung kommen sollen.

Beispielsweise aus der EP 0 776 223 B1 ist bereits eine derartige Vorrichtung bekannt. Hier ist ein Primärkreislauf für die zu behandelnde Flüssigkeit mit einem in diesen integrierten Filter vorhanden. An der Sekundärseite des Filters ist ein Sekundärkreislauf angeschlossen, in dem mindestens ein Adsorber angeordnet ist.

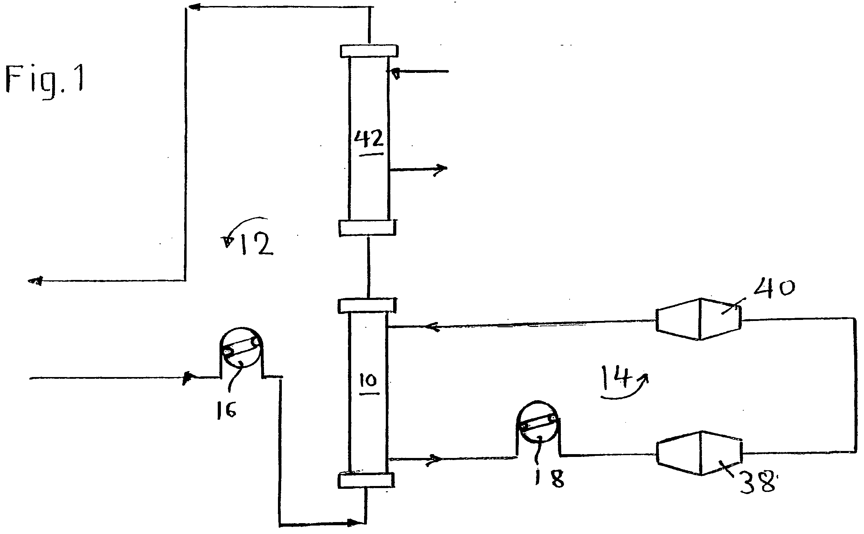

Eine entsprechende Vorrichtung wie in der Figur 1 beschrieben ist bekannt. Dort ist ein Primärkreislauf 12 gezeigt, in dem die Flüssigkeit des Primärkreislaufs, beispielsweise Blut, durch die Pumpe 16 zirkuliert wird. Im Primärkreislauf ist ein Filter 10 integriert. An der Sekundärseite des Filters 10 schließt ein Sekundärkreislauf 14 an, in welchem die Flüssigkeit des Sekundärkreislaufs über eine Rollenpumpe 18 volumenkonstant zirkuliert wird. In dem Kreislauf sind zwei Adsorberelemente 38 und 40 zur Reinigung der Flüssigkeit des Sekundärkreislaufs 14 integriert. Bei dieser Anordnung stellt sich im Hämofilter ein bestimmter Druckgradient ein, wobei es im oberen Teil des Filters (Eingang der Sekundärflüssigkeit) zu einer Infusion von Sekundärflüssigkeit in den Blutkreislauf und am unteren Ende (Ausgang der Sekundärflüssigkeit) zu einem Entzug von Flüssigkeit aus dem Blutkreislauf kommt. Die ausgetauschten Volumina gleichen sich aufgrund der Volumenkonstanz genau aus. Nachteilig ist es jedoch, dass die absolut ausgetauschte und damit gereinigte Flüssigkeitsmenge nicht genau definiert ist.

Während mit dem Filter 10 beispielsweise die Entgiftung von Blut bei schweren Leberversagen möglich ist, kann im Primärkreislauf, wie in der Figur 1 nach dem Stand der Technik gezeigt, zusätzlich ein Hämodialysator 42 angeordnet sein, mittels dem wasserlösliche Toxine aus dem Blut mit Hilfe einer Dialysemaschine über den extrakorporalen Blutkreislauf entfernt werden können.

Aus der DE 4 338 858 ist eine Vorrichtung bekannt, in dersen Sekundärkreislauf ein Adsorber sowie ein Expansionsgefäß angeordnet ist.

Aufgabe der Erfindung ist es, eine genaue Erfassung der ausgetauschten Volumina im Sekundärkreislauf zu ermöglichen.

Erfindungsgemäß wird diese Aufgabe durch die Kombination der Merkmale des Anspruchs 1 gelöst. Demnach wird im Sekundärkreislauf ein Expansionsgefäß integriert, in welchem Flüssigkeit des Sekundärkreislaufs diskontinuierlich aufgenommen und wieder abgegeben werden kann. Die Erfindung macht sich bei dieser Lösung das Prinzip von sogenannten "Einfachnadel"- oder "Single-Needle"-Systemen, wie sie beispielsweise aus der EP 0 472 480 B1 bekannt sind, zu Nutze. Bei derartigen Verfahren wird der Zugang zum Patienten durch eine einzige Kanüle hergestellt, durch die Blut abwechselnd angesaugt und zurückgegeben wird. Dies erfordert die Zwischenspeicherung von Blut in einem Expansionsgefäß. Im Rahmen der vorliegenden Erfindung wird kein "Einfachnadel"-System verwendet, sondern es wird hier im Sekundärkreislauf ein Expansionsgefäß integriert, in welches in einer ersten Phase Flüssigkeit durch den Filter angesaugt bzw. gedrückt wird. In einer zweiten, sich anschließenden Phase wird dann die Flüssigkeit aus dem Expansionsgefäß über den mindestens einen Filter an das Blut zurückgegeben. Da in dem Filter je nach Zyklus entweder eine Infusion in das Blut oder eine Filtration aus dem Blut stattfindet, und da die Förderraten im Sekundärkreislauf genau bekannt sind, können die ausgetauschten Flüssigkeitsmengen genau angegeben und damit bilanziert werden. Mit der hier vorgeschlagenen Vorrichtung kann ein bestimmter Filtratfluß vom Anwender vorgegeben werden. So kann hier ein wesentlich langsamerer Durchflußvolumenstrom gewählt werden, so daß die Verweilzeit der Sekundärflüssigkeit in den Blutbehandlungselementen, d. h. beispielsweise den Adsorbern größer ist, wodurch der Adsorbtionsgrad von Toxinen möglicherweise verbessert wird.

Weitere Einzelheiten und Vorteile der Erfindung ergeben sich aus den sich an den Hauptanspruch anschließenden Unteransprüchen.

Vorteilhaft können im Sekundärkreislauf Ventile zur Verteilung der Flüssigkeit des Sekundärkreislaufs beim diskontinuierlichen Befüllen und Entleeren des Expansionsgefäßes vorhanden sein.

Gemäß einer anderen vorteilhaften Ausgestaltung der Erfindung kann der Füllzustand des Expansionsgefäßes über einen Drucksensor erfaßbar sein.

Für die Pumpe des Sekundärkreislaufs kann auf Module aus sogenannten "Single-Needle"-Systemen zurückgegriffen werden, wobei der Drucksensor integriert ist. Im Primärkreislauf kann Blut und im Sekundärkreislauf Plasma zirkulieren. Bei dieser Anwendung kann als Filter vorzugsweise ein Hohlfasermembranfilter (z. B. auf Polysulfonbasis) eingesetzt werden. Dieser dient der Trennung des Plasmas vom Blut, wobei der Membranfilter für Albumin und Substanzen mit niedrigem Molekulargewicht durchlässig ist.

Zusätzlich kann vorteilhaft im Primärkreislauf ein Hämodialysator zur gleichzeitigen Dialysebehandlung integriert sein. Mit der Dialysebehandlung werden dann wasserlösliche Toxine aus dem Blut mit Hilfe der Dialysemaschine über den extrakorporalen Blutkreislauf entfernt. Bei einem Leberversagen können diese unlöslichen Toxine, die sich im Patientenblut anreichern und die an Eiweiße, meistens an Albumin, gebunden sind, durch den Sekundärkreis gemäß der vorliegenden Vorrichtung und hier insbesondere durch die Adsorbtion getrennt werden.

Weitere Merkmale, Einzelheiten und Vorteile der Erfindung ergeben sich aus einem in der Zeichnung dargestellten Ausführungsbeispiel. Es zeigen:
- Figur 1:: eine Vorrichtung zur Eliminierung von Substanzen aus Blut gemäß dem Stand der Technik und
- Figur 2:: eine Ausführungsform der erfindungsgemäßen Vorrichtung zur Eliminierung von Substanzen aus Blut.

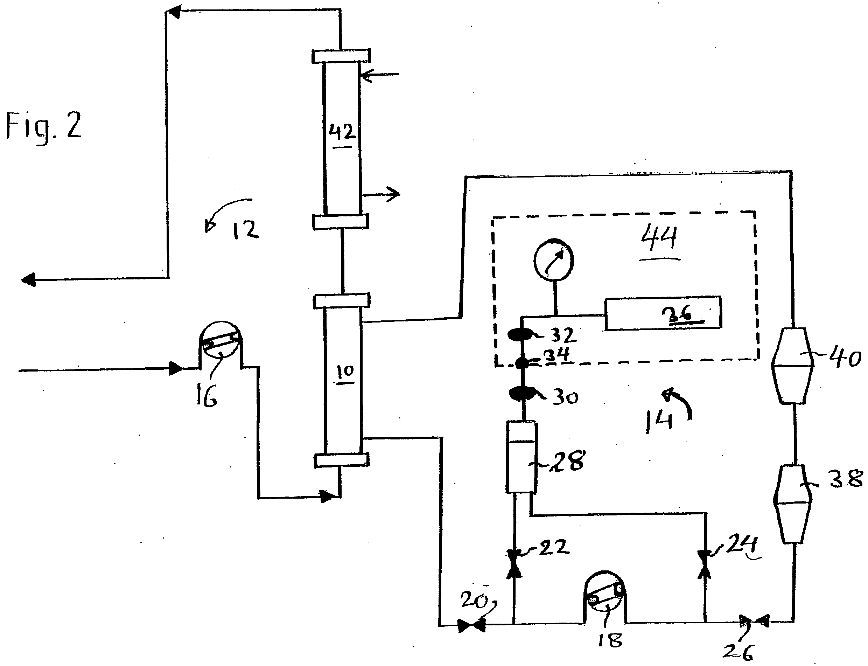

Die erfindungsgemäße Vorrichtung gemäß Figur 2 weist einen Primärkreislauf 12 auf, in welchem Patientenblut über eine Pumpe 16 gefördert wird. Im Primärkreislauf 12 ist ein Filter 10 integriert, das als Membranfilter aus Polysulfon ausgeführt ist. An den Membranfilter 10 schließt ein Sekundärkreislauf 14 an, bei dem eine als Rollenpumpe ausgeführte Pumpe 18 für die Förderung des in diesem Sekundärkreislauf geförderten Plasmas dient. Innerhalb des Sekundärkreislaufs sind zwei Adsorber 38 und 40 vorgesehen, über die eiweißgebundene und damit wasserunlösliche Toxine durch Adsorbtion aus dem Plasma entfernt werden können.

Im Sekundärkreislauf 14 ist ein Expansionsgefäß 28 integriert, in welchem Flüssigkeit des Sekundärkreislaufs 14, also Plasma, diskontinuierlich aufgenommen und wieder abgegeben werden kann. Zur Verteilung der Flüssigkeit des Sekundärkreislaufs beim diskontinuierlichen Befüllen und Entleeren des Expansionsgefäßes 28 sind Ventile 20, 22 und 24, 26 vorgesehen. Zwischen den Ventilen 20 und 22 einerseits und 24 und 26 andererseits ist, wie in Figur 2 dargestellt, die Pumpe 18 angeordnet. Das Expansionsgefäß steht weiterhin mit einem Drucksensor 44 in Verbindung, wobei der Drucksensor ein integrierter Bestandteil der Pumpe sein kann, wie sie bereits modulartig in "Single-Needle"-Systemen zum Einsatz kommen.

Hier sind Hydrophobfilter 30 und 32 vorhanden und ein Druckmeßanschluß 34. Mit 36 ist ein Ausgleichsgefäß bezeichnet. Mittels des Drucksensors 44 kann der Plasmafluß gesteuert werden.

Der Ablauf der einzelnen diskontinuierlichen Zyklen ergibt sich wie folgt. Zunächst sind die Ventile 20 und 24 geöffnet, während die Ventile 22 und 26 geschlossen sind. Die Pumpe 18 saugt in diesem Zustand über den Filter 10 frisches Plasma an und befördert es in das Expansionsgefäß 28, bis der obere Schaltdruck erreicht ist. Der Schaltdruck wird durch den Drucksensor 44 gemessen. Anschließend werden die Ventile 22 und 26 geöffnet, während die Ventile 20 und 24 geschlossen sind. In diesem Fall wird das Plasma aus dem Expansionsgefäß 28 gefördert und nach Durchströmen der Adsorber 38 und 40 dem Filter 10 und damit dem Primärkreislauf wieder zugeführt. Hierdurch kann eine genau definierte Filtratsvolumenstrommenge eingestellt werden. In einem gängigen System beträgt der Volumenstrom 20 bis 50 ml/min. Hiermit ist die Verweilzeit des Plasmas in den Adsorbern vergleichsweise größer. Dadurch kann die Adsorbtion von Toxinen verbessert werden.

Alternativ kann das System, wie es aus dem Stand der Technik bereits bekannt war, mit einem relativ hohen Rezirkulationsfluss auf der Plasmaseite betrieben werden. Hierzu werden die Ventile 20 und 26 geöffnet, während die Ventile 22 und 24 geschlossen werden. Hier kann im Vergleich zu den vorhergenannte Volumenströmen ein Plasmavolumenstrom in der Größenordnung von 300 ml/min. realisiert werden.

Der Filter 10 besteht aus einem Membranfilter aus Polysulfon, der für Albumin und Substanzen mit niedrigem Molekulargewicht durchlässig ist. Dieses dient zur Trennung des Patientenplasmas vom Blut. In dem Sekundärkreislauf wird, wie zuvor im einzelnen beschrieben, eine Plasmaseparation mittels Adsorbtion zur Entfernung der eiweißgebundenen Toxine durchgeführt.

Zusätzlich kann über einen Hämodialysator 42 im Primärkreislauf eine Dialysebehandlung durchgeführt werden, über welche die wasserlöslichen Toxine aus dem Blut mit Hilfe einer hier nicht näher dargestellten Dialysemaschine über den extrakorporalen Blutkreislauf entfernt werden.

## Patentansprüche

1. Vorrichtung zur Eliminierung von Substanzen aus Flüssigkeiten, insbesondere Blut, bestehend aus einem Primärkreislauf (12) für die zu behandelnde Flüssigkeit, einem in diesen integrierten Filter (10), an dessen Sekundärseite ein Sekundärkreislauf (14) angeschlossen ist, in dem mindestens ein Blutbehandlungselement, insbesondere ein Adsorber (38, 40), angeordnet sowie ein Expansionsgefäß (28) integriert ist, wobei im Primär- und Sekundärkreislauf jeweils Pumpen (16, 18) vorgesehen sind,
**dadurch gekennzeichnet, dass** das im Sekundärkreislauf (14) integrierte Expansionsgefäß (28) geeignet ist, über die Pumpe (18) des Sekundärkreislaufes (14), welche parallel zum Expansionsgefäß (28) angeordnet ist, Flüssigkeit vom Primärkreislauf (12) in den Sekundärkreislauf (14) diskontinuierlich aufzunehmen, wobei die Flüssigkeit nach Durchströmen des mindestens einen Adsorbers (38, 40) dem Filter (10) des Primärkreislaufes (12) und somit dem Primärkreislauf (12) wieder zurückführbar ist, und wobei die Pumpe (18) stromaufwärts des mindestens einen Absorbers (38, 40) angeordnet ist.

2. Vorrichtung nach Anspruch 1, **dadurch gekennzeichnet, dass** im Sekundärkreislauf (14) Ventile (20,22 bzw. 24,26) zur Verteilung der Flüssigkeit des Sekundärkreislaufs (14) beim diskontinuierlichen Befüllen und Entleeren des Expansionsgefäßes (28) vorhanden sind, wobei die Ventile (20, 22) und (24, 26) paarweise parallel zueinander angeordnet sind.

3. Vorrichtung nach einem der Ansprüche 1 oder 2, insbesondere nach Anspruch 2, **dadurch gekennzeichnet, dass** die Pumpe (18) geeignet ist, bei geöffneten Ventilen (20, 24) und geschlossenen Ventilen (22, 26) Flüssigkeit über den Filter (10) des Primärkreislaufes (12) anzusaugen und in das Expansionsgefäß (28) zu befördern und bei geöffneten Ventilen (22, 26) und geschlossenen Ventilen (20, 24) Flüssigkeit aus dem Expansionsgefäß (28) zu fördern, und nach Durchströmen des mindestens einen Absorbers (28, 40) dem Filter (10) zuzuführen.

4. Vorrichtung nach einem der vorhergehenden Ansprüche, **dadurch gekennzeichnet, dass** der Füllzustand des Expansionsgefäßes (28) über einen Drucksensor (44) erfassbar ist.

5. Vorrichtung nach Anspruch 4, **dadurch gekennzeichnet, dass** die Pumpe des Sekundärkreislaufs (14) und der Drucksensor (44) in einem Modul integriert sind.

6. Vorrichtung nach einem der vorhergehenden Ansprüche, **dadurch gekennzeichnet, dass** im Primärkreislauf (12) Blut und im Sekundärkreislauf (14) Plasma zirkulieren kann.

7. Vorrichtung nach Anspruch 6, **dadurch gekennzeichnet, dass** als Filter ein Membranfilter (10), vorzugsweise aus Polysulfon, eingesetzt wird.

8. Vorrichtung nach einem der vorhergehenden Ansprüche, **dadurch gekennzeichnet, dass** im Primärkreislauf (12) zusätzlich ein Hämodialysator (42) zur gleichzeitigen Dialysebehandlung integriert ist.

## Claims

1. An apparatus for the elimination of substances from liquids, in particular from blood, comprising a primary circuit (12) for the liquid to be treated, a filter (10) which is integrated therein and at whose secondary side a secondary circuit (14) is connected in which at least one blood treatment element, in particular an adsorber (38, 40), is arranged and in which an expansion vessel (28) is integrated, wherein respective pumps (16, 18) are provided in the primary and secondary circuits,
**characterized in that**
the expansion vessel (28) integrated in the secondary circuit (14) is suited for discontinuously taking up liquid from the primary circuit (12) into the secondary circuit (14) via the pump (18) of the secondary circuit (14) which is arranged in parallel with the expansion vessel (28), wherein the liquid can be returned to the filter (10) of the primary circuit (12) and thus to the primary circuit (12) after having flowed through the at least one adsorber (38, 40) and wherein the pump (18) is arranged upstream of the at least one absorber (38, 40).

2. An apparatus in accordance with claim 1, **characterized in that** valves (20, 22 or 24, 26) are present in the secondary circuit (14) for the distribution of the liquid of the secondary circuit (14) on the discontinuous filling and emptying of the expansion vessel (28), with the valves (20, 22) and (24, 26) being arranged in pairs in parallel with each other.

3. An apparatus in accordance with either of claims 1 or 2, in particular in accordance with claim 2, **characterized in that** the pump (18) is suited for sucking in liquid via the filter (10) of the primary circuit (12) and for conveying it into the expansion vessel (28) when the valves (20, 24) are open and the valves (22, 26) are closed and for conveying liquid out of the expansion vessel (28) when the valves (22, 26) are open and the valves (20, 24) are closed and for supplying it to the filter (10) after having flowed through the at least one absorber (28, 40).

4. An apparatus in accordance with any one of the preceding claims, **characterized in that** the filling level of the expansion vessel (28) can be detected via a pressure sensor (44).

5. An apparatus in accordance with claim 4, **characterized in that** the pump of the secondary circuit (14) and the pressure sensor (44) are integrated in a module.

6. An apparatus in accordance with any one of the preceding claims, **characterized in that** blood can circulate in the primary circuit (12) and plasma in the secondary circuit (14).

7. An apparatus in accordance with claim 6, **characterized in that** a membrane filter (10), preferably made from polysulfone, is used as the filter.

8. An apparatus in accordance with any one of the preceding claims, **characterized in that** a hemodialyzer (42) is additionally integrated in the primary circuit (12) for the simultaneous dialysis treatment.

## Revendications

1. Dispositif pour éliminer des substances hors de liquides, notamment du sang, constitué d'un circuit primaire (12) pour le liquide à traiter, d'un filtre (10) intégré dans celui-ci, au côté secondaire duquel est relié un circuit secondaire (14), dans lequel au moins un élément de traitement du sang, en particulier un adsorbant (38, 40), est disposé et un vase d'expansion (28) intégré, des pompes (16, 18) étant prévues respectivement dans le circuit primaire et secondaire, **caractérisé en ce que** le vase d'expansion (28) intégré dans le circuit secondaire (14) est adapté pour recevoir de manière discontinue du liquide du circuit primaire (12) dans le circuit secondaire (14) par le biais de la pompe (18) du circuit secondaire (14), qui est disposée parallèlement au vase d'expansion (28), le liquide pouvant être ramené de nouveau au filtre (10) du circuit primaire (12) et ainsi au circuit primaire (12) après avoir traversé l'au moins un adsorbant (38, 40), et la pompe (18) étant disposée en amont de l'au moins un absorbant (38, 40).

2. Dispositif selon la revendication 1, **caractérisé en ce que** des vannes (20, 22 ou 24, 26), destinées à distribuer le liquide du circuit secondaire (14) lors du remplissage et du vidage discontinus du vase d'expansion (28), se trouvent dans le circuit secondaire (14), les vannes (20, 22) et (24, 26) étant disposées par paires parallèles les unes aux autres.

3. Dispositif selon l'une des revendications 1 ou 2, en particulier selon la revendication 2, **caractérisé en ce que** la pompe (18) est adaptée pour, lorsque les vannes (20, 24) sont ouvertes et les vannes (22, 26) sont fermées, aspirer du liquide par le biais du filtre (10) du circuit primaire (12) et l'acheminer dans le vase d'expansion (28), et lorsque les vannes (22, 26) sont ouvertes et les vannes (20, 24) sont fermées, refouler du liquide hors du vase d'expansion (28), et l'alimenter dans le filtre (10) après avoir traversé l'au moins un absorbant (28, 40).

4. Dispositif selon l'une des revendications précédentes, **caractérisé en ce que** l'état de remplissage du vase d'expansion (28) peut être détecté par un capteur de pression (44).

5. Dispositif selon la revendication 4, **caractérisé en ce que** la pompe du circuit secondaire (14) et le capteur de pression (44) sont intégrés dans un module.

6. Dispositif selon l'une des revendications précédentes, **caractérisé en ce que** du sang peut circuler dans le circuit primaire (12) et du plasma dans le circuit secondaire (14).

7. Dispositif selon la revendication 6, **caractérisé en ce qu'**un filtre à membrane (10), de préférence en polysulfone, est utilisé comme filtre.

8. Dispositif selon l'une des revendications précédentes, **caractérisé en ce qu'**un hémodialyseur (42) est intégré en plus dans le circuit primaire (12) pour le traitement par dialyse simultané.
